# EUROPEAN PATENT APPLICATION

(11) **EP 3 834 761 A1**
(43) Date of publication of application: **16.06.2021**
(21) Application number: 19847768.9
(22) Date of filing: 06.08.2019
(51) Int. Cl.: A61B 18/18

(54) **MEDICAL TREATMENT INSTRUMENT**

(30) Priority: 06.08.2018 JP 2018147403
(71) Applicant: Saney Seiko Inc., Saitama 351-0024 (JP)
(72) Inventor: ISHIZEKI Yasunori, Asaka-shi, Saitama 351-0024 (JP); MANPUKU Yasuhiro, Asaka-shi, Saitama 351-0024 (JP); ISHIKAWA Hideki, Asaka-shi, Saitama 351-0024 (JP); MATSUZAKI Masahiro, Asaka-shi, Saitama 351-0024 (JP); OGIWARA Mariho, Asaka-shi, Saitama 351-0024 (JP)
(74) Representative: Schmidt, Christian
(86) International application number: PCT/JP2019/030807
(87) International publication number: WO 2020/031994

(57) **Abstract**

When tissue is irradiated with a microwave radiated from both electrodes, there is a problem of sparks. Further, when a ceramics material is used for the electrodes to eliminate the sparks, there is a problem that the ceramics is dropped during operation. Provided is a medical treatment instrument that solves those problems. When the medical treatment instrument adopts structure in which microwave application antennas are in contact with each other under a state in which a first electrode (2) and a second electrode (5) are closed or structure in which microwave reception antennas are in contact with each other under the state in which the first electrode (2) and the second electrode (5) are closed, tissue can be efficiently irradiated with a microwave radiated from both electrodes (2 and 5).

## Description

### Technical Field

The present invention relates to a medical treatment instrument which is capable of radiating a microwave from both electrodes.

The present application claims priority from Japanese Patent Application No. 2018-147403, the disclosure of which is incorporated herein by reference.

### Background Art

### (Device Using Microwave)

There has been known that a microwave can coagulate (immobilize) biological tissue such as digestive organs, a liver, a bladder, a prostate, a uterus, blood vessels, or intestinal tracts at low temperature. Then, various devices for assisting surgery through use of the microwave have been developed.

### (Related Art)

As the devices using a microwave, the following plurality of devices have been reported.

In Patent Literature 1, there is disclosed "a power distribution circuit including two distribution lines which are branched into two in the vicinity of an input terminal having electrical continuity to an input line and have electrical continuity to corresponding output lines at respective output terminals, the power distribution circuit including: two connection lines having electrical continuity to the output terminals of the two distribution lines, respectively; an isolation resistor arranged between the two connection lines; and a stub provided at a branch portion of the two distribution lines."

In Patent Literature 1, the distribution circuit for a microwave is disclosed, but the configuration of the medical treatment instrument according to the present invention is not disclosed.

In Patent Literature 2, there is disclosed "a microwave surgical instrument including: a surgical instrument main body including an electrode portion for irradiating biological tissue with a microwave; a microwave oscillator which is provided in the surgical instrument main body, and is configured to oscillate a microwave; and an amplifier which is provided in the surgical instrument main body, and is connected between the electrode portion and the microwave oscillator, the amplifier being configured to amplify the microwave from the microwave oscillator and send the amplified microwave to the electrode portion, the microwave surgical instrument further including: a variable output matching circuit which is connected between the amplifier and the electrode portion, and is configured to match an output impedance of the amplifier and an impedance of the biological tissue with each other; a detection circuit configured to separately detect reflected power and incident power given between the amplifier and the electrode portion; and control means for controlling the variable output matching circuit based on values of the incident power and the reflected power detected by the detection circuit."

In Patent Literature 2, the variable output matching circuit for matching the output impedance of the amplifier and the impedance of the biological tissue with each other is disclosed, but the feature that the microwave can be radiated from both electrodes of the medical treatment instrument according to the present invention and the configuration of the electrodes are not disclosed.

In Patent Literature 3, there is disclosed "electrosurgical forceps including: a pair of jaw elements pivotable relative to each other to open and close a gap therebetween; a first transmission line structure mounted in one of the pair of jaw elements adjacent to the gap; a second transmission line structure mounted in the other one of the pair of jaw elements adjacent to the gap opposite the first transmission line structure; a coaxial cable for conveying microwave frequency energy; and a power splitter at a distal end of the coaxial cable, the power splitter being arranged to divide the microwave frequency energy conveyed by the coaxial cable between the first transmission line structure and the second transmission line structure, in which each of the first transmission line structure and the second transmission line structure consists of an unbalanced lossy transmission line to support the microwave energy as a travelling wave, and in which each of the first transmission line structure and the second transmission line structure has an electrical length along the travelling wave that is non-resonant for the microwave energy."

In Patent Literature 3, the feature that the microwave can be radiated from both electrodes is disclosed, but the configuration of the electrodes of the medical treatment instrument according to the present invention is not disclosed.

Consequently, none of the patent literatures discloses or suggests the electrode structure of the present invention that allows efficient radiation of a microwave from both electrodes.

### Citation List

### Patent Literature

[PTL 1] JP 11-330813 A
[PTL 2] JP 2012-115384 A
[PTL 3] JP 2016-533862 A1

### Summary of Invention

### Technical Problem

There is difficulty in efficiently irradiating tissue with a microwave radiated from both electrodes, and there arises a problem of sparks. Further, when a ceramic material is used for the electrodes to eliminate the sparks, there arises a problem that the ceramic is dropped during operation.

### Solution to Problem

As a result of extensive studies conducted to solve the problems described above, the inventors of the present invention found that, when the structure in which microwave application antennas are in contact with each other under a state in which a first electrode and a second electrode are closed or the structure in which microwave reception antennas are in contact with each other under the state in which the first electrode and the second electrode are closed is adopted, tissue can be efficiently irradiated with a microwave radiated from both electrodes. Based on the finding, the inventors achieved the present invention.

That is, the present invention includes the following aspects.
1. A medical treatment instrument including a distal end portion,
   the distal end portion including:
   a first electrode including a microwave application antenna 1 and a microwave reception antenna 1;
   a second electrode which is arranged to be opposed to the first electrode, and includes a microwave application antenna 2 and a microwave reception antenna 2; and
   a coaxial cable including a center conductor and an outer conductor, the center conductor being directly or indirectly connected to the microwave application antenna 1 and the microwave application antenna 2, the outer conductor being directly or indirectly connected to the microwave reception antenna 1 and the microwave reception antenna 2,
   wherein the medical treatment instrument has structure in which the microwave application antenna 1 and the microwave application antenna 2 are in contact with each other under a state in which the first electrode and the second electrode are closed or structure in which the microwave reception antenna 1 and the microwave reception antenna 2 are in contact with each other under the state in which the first electrode and the second electrode are closed, and
   wherein a microwave is allowed to be radiated from both the first electrode and the second electrode.
2. The medical treatment instrument according to the above-mentioned item 1, wherein the center conductor has a distal end having a shape that is branched into two legs, and the microwave application antenna 1 and the microwave application antenna 2 are directly or indirectly connected to the branched two legs of the center conductor.
3. The medical treatment instrument according to the above-mentioned item 1, wherein the microwave application antenna 1 and the microwave application antenna 2 are connected to the outer conductor through intermediation of a pin.
4. The medical treatment instrument according to any one of the above-mentioned items 1 to 3, wherein, in the first electrode, the microwave application antenna 1 and the microwave reception antenna 1 are installed through intermediation of an insulator or a dielectric, and in the second electrode, the microwave application antenna 2 and the microwave reception antenna 2 are installed through intermediation of an insulator or a dielectric.
5. The medical treatment instrument according to any one of the above-mentioned items 1 to 4, wherein a distal end of the microwave application antenna 1 and a distal end of the microwave reception antenna 1 are connected to each other, and/or a distal end of the microwave application antenna 2 and a distal end of the microwave reception antenna 2 are connected to each other.
6. The medical treatment instrument according to any one of the above-mentioned items 1 to 4, wherein a distal end of the microwave application antenna 1 and a distal end of the microwave reception antenna 1 are connected to each other through intermediation of a pin, and/or a distal end of the microwave application antenna 2 and a distal end of the microwave reception antenna 2 are connected to each other through intermediation of a pin.
7. The medical treatment instrument according to any one of the above-mentioned items 1 to 6, wherein the first electrode is a fixed electrode, and the second electrode is a movable electrode.
8. The medical treatment instrument according to any one of the above-mentioned items 1 to 7, further including:
   a shaft portion including the coaxial cable; and
   a grip portion including a connector to which the coaxial cable is connected,
   wherein rotation of the connector allows the distal end portion and the shaft portion to rotate.
9. The medical treatment instrument according to the above-mentioned item 8, further including a shaft rotating knob installed on the shaft portion.
10. The medical treatment instrument according to any one of the above-mentioned items 1 to 9, wherein the first electrode and the second electrode are formed without use of ceramic.
11. A medical treatment instrument including a distal end portion,
   the distal end portion including:
   a first electrode including a microwave application antenna 1 and a microwave reception antenna 1;
   a second electrode which is arranged to be opposed to the first electrode, and includes a microwave application antenna 2 and a microwave reception antenna 2; and
   a coaxial cable including a center conductor and an outer conductor, the center conductor being directly or indirectly connected to the microwave application antenna 1 and the microwave application antenna 2, the outer conductor being directly or indirectly connected to the microwave reception antenna 1 and the microwave reception antenna 2,
   wherein, in the first electrode, the microwave application antenna 1 and the microwave reception antenna 1 are installed through intermediation of an insulator or a dielectric, and in the second electrode, the microwave application antenna 2 and the microwave reception antenna 2 are installed through intermediation of an insulator or a dielectric,
   wherein the medical treatment instrument has structure in which the microwave application antenna 1 and the microwave application antenna 2 are in contact with each other under a state in which the first electrode and the second electrode are closed, and
   wherein a microwave is allowed to be radiated from both the first electrode and the second electrode.
12. The medical treatment instrument according to the above-mentioned item 11, wherein the first electrode and the second electrode are formed without use of ceramic.

### Advantageous Effect of Invention

With the medical treatment instrument of the present invention, tissue can be efficiently irradiated with the microwave radiated from both electrodes.

### Brief Description of Drawings

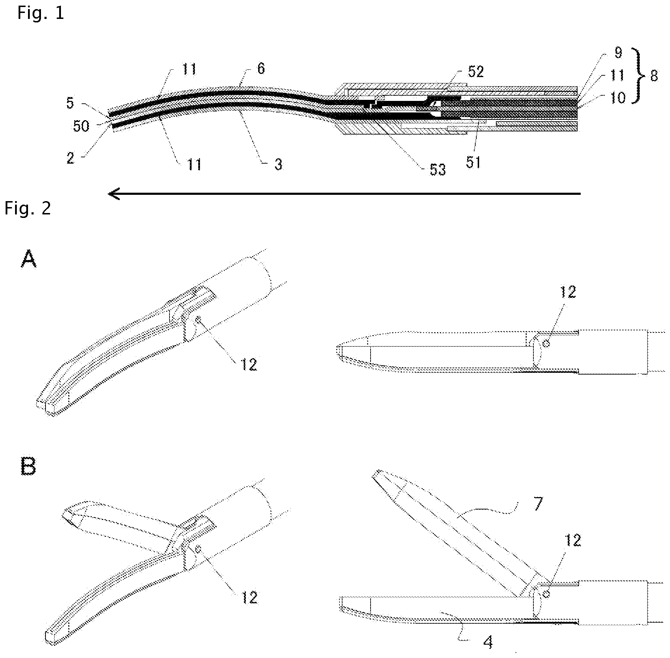

FIG. 1 is a sectional view for illustrating a distal end portion of a medical treatment instrument according to the present invention (the structure in which microwave application antennas are in contact with each other) as seen from above (the direction of the arrow (long-axis direction) corresponds to a distal end side, and the opposite direction corresponds to a terminal end side).
FIGS. 2 are views for illustrating an opened state and a closed state of both electrodes at the distal end portion of the medical treatment instrument according to the present invention. A: The state in which the electrodes are closed. B:The state in which the electrodes are opened.

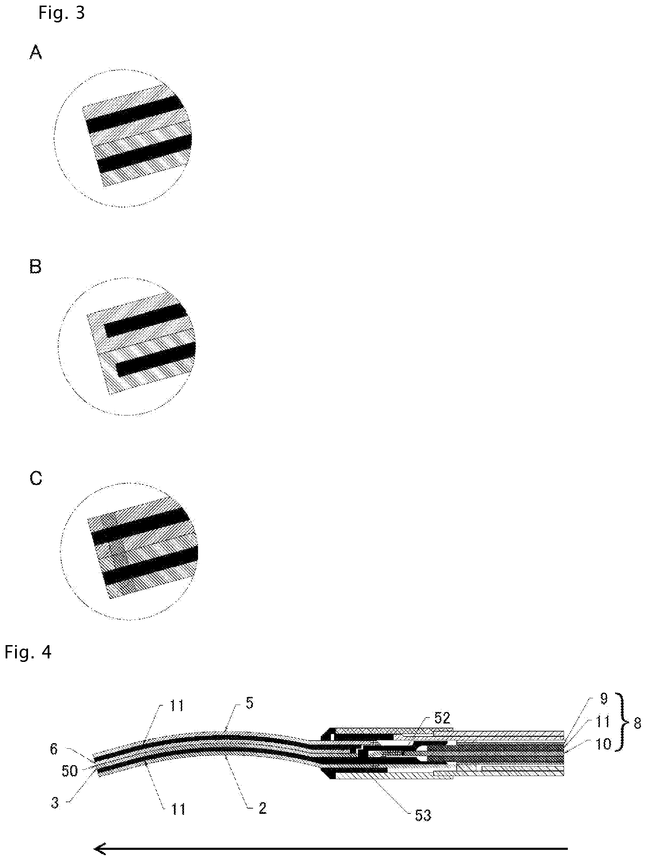

FIGS. 3 are distal end views for illustrating the distal end portion of the medical treatment instrument according to the present invention. A: In both electrodes, a microwave application antenna and a microwave reception antenna are separated from each other. B: In both electrodes, the microwave application antenna and the microwave reception antenna are integrated with each other (connected to each other). C: In both electrodes, the microwave application antenna and the microwave reception antenna are connected to each other through intermediation of a pin.
FIG. 4 is a sectional view for illustrating a distal end portion of a medical treatment instrument according to another mode of the present invention (the structure in which microwave reception antennas are in contact with each other) as seen from above (the direction of the arrow (long-axis direction) corresponds to the distal end side, and the opposite direction corresponds to the terminal end side).

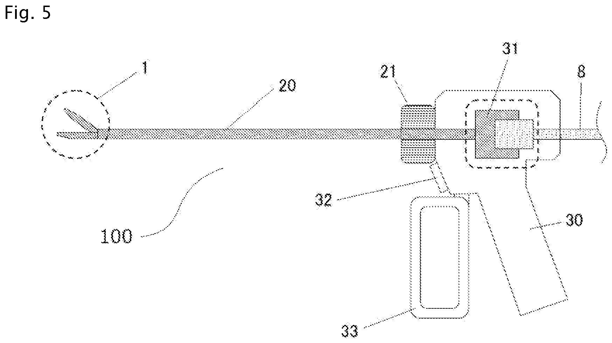

FIG. 5 is an overall view of the medical treatment instrument according to the present invention.

### Description of Embodiments

Hereinafter, the present invention is described with reference to the drawings. However, the present invention is not limited to medical treatment illustrated in the drawings.

### (Medical Treatment Instrument)

A medical treatment instrument (100) according to the present invention relates to a microwave radiation instrument (in particular, microwave radiation surgical instrument) capable of irradiating tissue with a microwave from both electrodes (see FIG. 1 to FIG. 5).

The medical treatment instrument (100) according to the present invention includes distal end portion (1) comprising at least the following components.

A first electrode (4) including a microwave application antenna 1 (2) and a microwave reception antenna 1 (3).

A second electrode (7) which is arranged to be opposed to the first electrode (4), and includes a microwave application antenna 2 (5) and a microwave reception antenna 2 (6).

A coaxial cable (8) including a center conductor (10) and an outer conductor (9). The center conductor (10) is directly or indirectly connected to the microwave application antenna 1 (2) and the microwave application antenna 2 (5), and the outer conductor (9) is directly or indirectly connected to the microwave reception antenna 1 (3) and the microwave reception antenna 2 (6).

The medical treatment instrument (100) according to the present invention has structure in which the microwave application antenna 1 (2) and the microwave application antenna 2 (5) are in contact with each other under a state in which the first electrode (4) and the second electrode (7) are closed or structure in which the microwave reception antenna 1 (3) and the microwave reception antenna 2 (6) are in contact with each other under the state in which the first electrode (4) and the second electrode (7) are closed (see FIG. 2A), and a microwave is allowed to be radiated from both the first electrode (4) and the second electrode (7).

As an example of a method of connecting the microwave reception antenna 1 (3) and the microwave reception antenna 2 (6) to the outer conductor (9), the microwave reception antenna 1 (3) and the microwave reception antenna 2 (6) are connected to the outer conductor (9) through intermediation of a pin (12). With this configuration, both or one of the microwave reception antenna 1 (3) and the microwave reception antenna 2 (6) is movable. Therefore, the first electrode (4) and/or the second electrode (7) is movable.

As an example of the pin (12) in the present invention, although not particularly limited, the pin (12) may have any shape as long as two or more members can be directly or indirectly coupled to each other, or two or more members are rotatable about the pin (12).

As an example of a method of connecting the microwave application antenna 1 (2) and the microwave application antenna 2 (5) to the center conductor (10), the center conductor (10) has a distal end having a two-legged fork shape, that is, a shape that is branched into two legs, and the microwave application antenna 1 (2) and the microwave application antenna 2 (5) are directly or indirectly connected to the branched two legs of the center conductor (10), respectively. The microwave application antenna 1 (2) and the microwave application antenna 2 (5) are separated from the microwave reception antenna 1 (3) and the microwave reception antenna 2 (6), respectively, through intermediation of a dielectric (insulator) (11).

The medical treatment instrument (100) according to the present invention is not particularly limited as long as the medical treatment instrument (100) is movable such that tissue can be gripped in a gap defined between both electrodes which are the first electrode (4) and the second electrode (7), and examples thereof may include forceps and tweezers (see FIGS. 2). As an example of the forceps, any publicly known forceps can be used as the forceps used in the present invention. Examples of the forceps used in the present invention may include Kelly forceps, Kocher forceps, Pean forceps, and Allis forceps. However, the forceps are not particularly limited.

### (Irradiation Microwave)

A frequency of microwave to be transmitted to the coaxial cable (8) of the medical treatment instrument (100) according to the present invention is not particularly limited, but is from 300 MHz to 300 GHz (wavelength: from 1 m to 1 mm), preferably from 0.9 GHz to 30 GHz. A transmission method can easily be achieved by any publicly known method, for example, a method of connecting the medical treatment instrument (100) to a publicly known microwave oscillator configured to oscillate a microwave or a method of providing the oscillator in the medical treatment instrument (101).

Power used in the present invention is from 0.1 W to 200 W, preferably from 1.0 W to 80 W.

### (Coaxial Cable)

The coaxial cable (8) used in the present invention is formed of, for example, a center conductor, a shield tube, and an earth pipe or a braided copper wire. The center conductor is an electric conductor made of copper. The shield tube is an insulator or a dielectric (which is made of, for example, Teflon (trademark) or polyethylene) configured to cover the center conductor. The earth pipe or the braided copper wire is an outer conductor (electric conductor) made of, for example, copper, stainless steel, or brass.

The coaxial cable (8) may be a publicly known semi-rigid coaxial cable.

### (Antenna)

The microwave application antenna 1 (2) and the microwave application antenna 2 (5) of the present invention are not particularly limited as long as the microwave application antenna 1 (2) and the microwave application antenna 2 (5) are made of a material that enables supply of a microwave. A conductive material, such as silver, copper, gold, iron, titanium, stainless steel, phosphor bronze, or brass, may be widely used. Suitable examples thereof include silver, copper, gold, stainless steel, and brass.

The microwave reception antenna 1 (3) and the microwave reception antenna 2 (6) of the present invention are not particularly limited as long as the microwave reception antenna 1 (3) and the microwave reception antenna 2 (6) are made of a material that enables reception of a microwave. A conductive material, such as silver, copper, gold, iron, titanium, stainless steel, phosphor bronze, or brass, may be widely used. Suitable examples thereof include silver, copper, gold, stainless steel, and brass.

A shape of the antenna is not particularly limited. Examples of the shape of the antenna include a circular cone, a triangular pyramid, a quadrangular pyramid, a column, a quadrangular prism, a triangular prism, a sphere, a cube, a cuboid, a tooth shape, a cylindrical shape, a rod shape, and a sawtooth shape. Further, for an inner surface (in particular, contact surface with respect to tissue) of the antenna, shapes such as a planar shape and a protrusion/recess shape are widely applicable.

As a specific configuration of the microwave application antenna of the present invention, the quadrangular prism may be adopted, and the contact surface with respect to tissue may have the sawtooth shape so that tissue is less liable to slip. As a specific configuration of the microwave reception antenna, the quadrangular prism may be adopted, and the contact surface with respect to tissue may have the sawtooth shape so that tissue is less liable to slip.

### (Electrode)

The first electrode (4) of the present invention includes the microwave application antenna 1 (2) and the microwave reception antenna 1 (3). The second electrode (7) of the present invention includes the microwave application antenna 2 (5) and the microwave reception antenna 2 (6).

Further, the first electrode (4) and the second electrode (7) are not particularly limited as long as the first electrode (4) and the second electrode (7) are movable so as to be capable of gripping tissue in the gap defined between both electrodes (in particular, forceps) and further have a coagulating function and/or a cutting function as needed. Further, any one or both of the first electrode (4) and the second electrode (7) may be a fixed electrode or a movable electrode.

It is more preferred that the electrodes each be partially or entirely subjected to coating that causes coagulated tissue to be less liable to adhere to the electrodes during contact with the tissue. The coating is performed with, for example, gold or a Teflon-based member. With this, processing of coagulation and cutting can be successively performed without causing adhesion of coagulated tissue. It is preferred that the electrodes of the present invention be formed without use of a ceramic material.

A configuration of the distal end portions of the first electrode (4) and/or the second electrode (7) of the present invention for efficient radiation of a microwave is described below.

In both electrodes, the microwave application antenna (2, 5) and the microwave reception antenna (3, 6) are separated from each other (preferably, separated in the range of from the terminal end to the distal end) (see FIG. 3A).

In both electrodes, the microwave application antenna (2, 5) and the microwave reception antenna (3, 6) are integrated with each other (connected to each other, preferably, the microwave application antenna (2, 5) and the microwave reception antenna (3, 6) are integrated with each other on the distal end side) (see FIG. 3B).

As examples of the integration, the microwave application antenna (2, 5) and the microwave reception antenna (3, 6) may be manufactured as an integrated component, or distal end portions thereof may have a material that does not affect the microwave radiation and is applied through vapor deposition.

In both electrodes, the microwave application antenna (2, 5) and the microwave reception antenna (3, 6) are connected to each other through intermediation of, for example, the pin (12) (see FIG. 3C).

In the first electrode (4), the microwave application antenna 1 (2) and the microwave reception antenna 1 (3) are installed through intermediation of the insulator (dielectric) (11). For example, the insulator (dielectric) (11) has such a shape that allows the insulator (dielectric) (11) to be sandwiched between the microwave application antenna 1 (2) and the microwave reception antenna 1 (3) (see FIG. 1). With this configuration, the microwave application antenna 1 (2) and the microwave reception antenna 1 (3) are partially or entirely insulated.

In the second electrode (7), the microwave application antenna 2 (5) and the microwave reception antenna 2 (6) are installed through intermediation of the insulator (dielectric) (11). More specifically, the insulator (dielectric) (11) has such a shape that allows the insulator (dielectric) (11) to be sandwiched between the microwave application antenna 2 (5) and the microwave reception antenna 2 (6) (see FIG. 1). With this configuration, the microwave application antenna 2 (5) and the microwave reception antenna 2 (6) are partially or entirely insulated.

Examples of the insulator or dielectric may include a PEEK resin, Teflon (trademark), and ceramic.

### (Shaft Portion)

A shaft portion (20) of the present invention (see FIG. 5) may have the same structure as that of a shaft portion of a publicly known medical instrument, but it is preferred that the shaft portion (20) include the coaxial cable (8). Further, the shaft portion (20) may include a shaft rotating knob (21). Through rotation of the shaft rotating knob (21), the distal end portion (1) and the shaft portion (20) can be rotated.

### (Grip Portion)

A grip portion (30) of the present invention (see FIG. 5) may have the same structure as that of a grip portion of a publicly known medical instrument. The grip portion (30) may include a connector (31), a microwave radiation switch (32), and an electrode moving switch (33).

The connector (31) is connected to the coaxial cable (8) so that rotation of the connector (31) allows the distal end portion (1) and the shaft portion (20) to rotate.

The microwave radiation switch (32) is capable of not only turning on and off the microwave radiation but also adjusting the intensity of the microwave radiation.

The electrode moving switch (33) is capable of performing an operation of opening and closing both electrodes.

The present invention is specifically described below by way of Examples, but the present invention is not limited to Examples.

### Example 1

(Example 1 of Medical Treatment Instrument according to the Present Invention)

In Example 1, there is given a medical treatment instrument having the structure in which the microwave application antennas are in contact with each other as illustrated in FIG. 1.

Stainless steel is used for each of the antennas, and a PEEK resin is used for the insulator (dielectric) (11). In both electrodes, the microwave application antenna and the microwave reception antenna are separated (see FIG. 3A).

It was confirmed that, through coagulation of a liver of a pig with use of the medical treatment instrument of Example 1, the medical treatment instrument of Example 1 was able to suppress sparks and evenly radiate a microwave.

### Example 2

### (Example 2 of Medical Treatment Instrument according to the Present Invention)

In Example 2, there is given a medical treatment instrument having the structure in which the microwave reception antennas are in contact with each other as illustrated in FIG. 4.

Stainless steel is used for each of the antennas, and a PEEK resin is used for the insulator (dielectric) (11). In both electrodes, the microwave application antenna and the microwave reception antenna are separated (see FIG. 3A).

The medical treatment instrument according to the present invention has one or more of the following effects.
(1) Tissue can be efficiently irradiated with a microwave radiated from both electrodes.
(2) Sparks can be suppressed without use of the ceramic material for both electrodes.
(3) A microwave can be substantially evenly radiated from surfaces of the microwave application antennas.

### Industrial Applicability

According to the present invention, a medical treatment instrument capable of efficiently irradiating tissue with a microwave radiated from both electrodes can be provided.

### Reference Signs List

100: medical treatment instrument
1: distal end portion
2: microwave application antenna 1
3: microwave reception antenna 1
4: first electrode (fixed electrode)
5: microwave application antenna 2
6: microwave reception antenna 2
7: second electrode (movable electrode)
8: coaxial cable
9: outer conductor
10: center conductor
11: dielectric (insulator)
12: pin
20: shaft portion
21: shaft rotating knob
30: grip portion
31: connector
32: microwave radiation switch
33: electrode moving switch
50: mating surfaces of upper and lower blades
51: connection portion between outer conductor of coaxial cable and outer conductor of electrode
52: connection portion between center conductor of coaxial cable and center conductor of electrode
53: portion at which antennas are internally in contact with each other and at which center conductor is branched

## Claims

1. A medical treatment instrument comprising a distal end portion,
the distal end portion including:
a first electrode including a microwave application antenna 1 and a microwave reception antenna 1;
a second electrode which is arranged to be opposed to the first electrode, and includes a microwave application antenna 2 and a microwave reception antenna 2; and
a coaxial cable including a center conductor and an outer conductor, the center conductor being directly or indirectly connected to the microwave application antenna 1 and the microwave application antenna 2, the outer conductor being directly or indirectly connected to the microwave reception antenna 1 and the microwave reception antenna 2,
wherein the medical treatment instrument has structure in which the microwave application antenna 1 and the microwave application antenna 2 are in contact with each other under a state in which the first electrode and the second electrode are closed or structure in which the microwave reception antenna 1 and the microwave reception antenna 2 are in contact with each other under the state in which the first electrode and the second electrode are closed, and
wherein a microwave is allowed to be radiated from both the first electrode and the second electrode.

2. The medical treatment instrument according to claim 1, wherein the center conductor has a distal end having a shape that is branched into two legs, and the microwave application antenna 1 and the microwave application antenna 2 are directly or indirectly connected to the branched two legs of the center conductor.

3. The medical treatment instrument according to claim 1, wherein the microwave application antenna 1 and the microwave application antenna 2 are connected to the outer conductor through intermediation of a pin.

4. The medical treatment instrument according to any one of claims 1 to 3, wherein, in the first electrode, the microwave application antenna 1 and the microwave reception antenna 1 are installed through intermediation of an insulator or a dielectric, and in the second electrode, the microwave application antenna 2 and the microwave reception antenna 2 are installed through intermediation of an insulator or a dielectric.

5. The medical treatment instrument according to any one of claims 1 to 4, wherein a distal end of the microwave application antenna 1 and a distal end of the microwave reception antenna 1 are connected to each other, and/or a distal end of the microwave application antenna 2 and a distal end of the microwave reception antenna 2 are connected to each other.

6. The medical treatment instrument according to any one of claims 1 to 4, wherein a distal end of the microwave application antenna 1 and a distal end of the microwave reception antenna 1 are connected to each other through intermediation of a pin, and/or a distal end of the microwave application antenna 2 and a distal end of the microwave reception antenna 2 are connected to each other through intermediation of a pin.

7. The medical treatment instrument according to any one of claims 1 to 6, wherein the first electrode is a fixed electrode, and the second electrode is a movable electrode.

8. The medical treatment instrument according to any one of claims 1 to 7, further comprising:
a shaft portion including the coaxial cable; and
a grip portion including a connector to which the coaxial cable is connected,
wherein rotation of the connector allows the distal end portion and the shaft portion to rotate.

9. The medical treatment instrument according to claim 8, further comprising a shaft rotating knob installed on the shaft portion.

10. The medical treatment instrument according to any one of claims 1 to 9, wherein the first electrode and the second electrode are formed without use of ceramic.

11. A medical treatment instrument comprising a distal end portion,
the distal end portion including:
a first electrode including a microwave application antenna 1 and a microwave reception antenna 1;
a second electrode which is arranged to be opposed to the first electrode, and includes a microwave application antenna 2 and a microwave reception antenna 2; and
a coaxial cable including a center conductor and an outer conductor, the center conductor being directly or indirectly connected to the microwave application antenna 1 and the microwave application antenna 2, the outer conductor being directly or indirectly connected to the microwave reception antenna 1 and the microwave reception antenna 2,
wherein, in the first electrode, the microwave application antenna 1 and the microwave reception antenna 1 are installed through intermediation of an insulator or a dielectric, and in the second electrode, the microwave application antenna 2 and the microwave reception antenna 2 are installed through intermediation of an insulator or a dielectric,
wherein the medical treatment instrument has structure in which the microwave application antenna 1 and the microwave application antenna 2 are in contact with each other under a state in which the first electrode and the second electrode are closed, and
wherein a microwave is allowed to be radiated from both the first electrode and the second electrode.

12. The medical treatment instrument according to claim 11, wherein the first electrode and the second electrode are formed without use of ceramic.
